# EUROPEAN PATENT APPLICATION

(11) **EP 4 835 488 A1**
(43) Date of publication of application: **04.11.2026**
(21) Application number: 24912778.8
(22) Date of filing: 24.12.2024
(51) Int. Cl.: G01N 35/10, G01N 37/00

(54) **LIQUID SAMPLE ANALYSIS SYSTEM AND MICROCHIP**

(30) Priority: 28.12.2023 JP 2023222269
(71) Applicant: ZACROS Corporation, Tokyo 112-0002 (JP)
(72) Inventor: SAKAI, Kazuo, Tokyo 112-0002 (JP); KAYANO, Makito, Tokyo 112-0002 (JP); WADA, Tomoko, Tokyo 112-0002 (JP); HOSOKAWA, Kazuya, Tokyo 112-0002 (JP)
(74) Representative: Watermeyer, Nicholas
(86) International application number: PCT/JP2024/045590
(87) International publication number: WO 2025/142891

(57) **Abstract**

A liquid sample analysis system comprises a pipette tip for aspirating and dispensing a liquid sample, and a dispensing pipette having the pipette tip attached at its distal end and having a space for accommodating the liquid sample aspirated through the pipette tip. The system further comprises a microchip having a connection portion for holding the dispensing pipette with the pipette tip attached, and a first flow channel formed therein for allowing the liquid sample introduced from the dispensing pipette to flow into a measurement portion for measuring a property of the liquid sample. The system further comprises a control unit connected to the dispensing pipette, which controls the flow rate of the liquid sample aspirated and dispensed through the pipette tip, and controls the delivery of the liquid sample introduced through the first flow channel to the measurement portion.

## Description

### Technical Field

The present invention relates to a liquid sample analysis system and a microchip.

### Background Art

Conventionally, in the medical field, biochemical field, and the like, tests and experiments have been conducted in which small amounts of liquid samples such as reagents and specimens are introduced into microfluidic chips or the like to cause reactions (see, for example, Patent Document 1). A liquid sample used in such reactions is introduced into a microfluidic chip using, for example, a dispensing pipette (also simply referred to as a pipette) having a pipette tip attached to a distal end thereof, the pipette tip being configured to aspirate and dispense the liquid sample.

### Prior Art Document

### Patent Document

Patent Document 1: International Publication No. WO 2009/069656

### Summary of the Invention

When a liquid sample used for testing or the like is present in a small amount, the stability of liquid delivery of the liquid sample introduced in the form of droplets through a pipette tip becomes a challenge. The present invention has been made in view of such circumstances, and an object thereof is to provide a technology for ensuring stability of liquid delivery of a liquid sample introduced through a pipette tip.

In order to achieve the above object, one embodiment of the present invention adopts the following configuration. That is, a liquid sample analysis system according to one embodiment comprises: a pipette tip configured to aspirate and dispense a liquid sample; a dispensing pipette having the pipette tip attached to a distal end thereof and having a space for accommodating the liquid sample aspirated through the pipette tip; a microchip having a connection portion configured to hold the dispensing pipette and the pipette tip attached thereto, and having a first flow channel formed therein through which the liquid sample introduced from the dispensing pipette flows into a measurement portion for measuring a property of the liquid sample; and a control unit connected to the dispensing pipette, configured to control a flow rate of the liquid sample aspirated and dispensed through the pipette tip, and configured to control delivery of the introduced liquid sample to the measurement portion through the first flow channel.

According to such a feature, the stability of liquid delivery of the liquid sample introduced through the pipette tip can be ensured.

Here, the microchip may comprise a filter configured to maintain a venting pressure of the liquid sample delivered to the measurement portion through the first flow channel within a predetermined range. Furthermore, the microchip may comprise a vent hole connected to the measurement portion via a second flow channel, and a sensor configured to detect that the delivered liquid sample has reached a predetermined region between the measurement portion and the vent hole, and the control unit may control the mechanism for dispensing the liquid sample from the dispensing pipette so as to stop dispensing of the liquid sample accommodated in the dispensing pipette, based on a detection signal output from the sensor indicating that the delivered liquid sample has reached the predetermined region.

Another embodiment of the present invention is a microchip comprising: a connection portion configured to hold a dispensing pipette having a pipette tip attached to a distal end thereof, the pipette tip being configured to aspirate and dispense a liquid sample, and the dispensing pipette having a space for accommodating the liquid sample aspirated through the pipette tip; a flow channel through which the liquid sample introduced from the dispensing pipette held by the connection portion flows into a measurement portion for measuring a property of the liquid sample; and a filter configured to maintain a venting pressure of the liquid sample delivered to the measurement portion through the flow channel within a predetermined range.

Even in such an embodiment, the stability of liquid delivery of the liquid sample introduced through the pipette tip can be ensured.

Another embodiment of the present invention is a microchip comprising: a connection portion configured to hold a dispensing pipette having a pipette tip attached to a distal end thereof, the pipette tip being configured to aspirate and dispense a liquid sample, and the dispensing pipette having a space for accommodating the liquid sample aspirated through the pipette tip; a first flow channel through which the liquid sample introduced from the dispensing pipette held by the connection portion flows into a measurement portion for measuring a property of the liquid sample; a vent hole connected to the measurement portion via a second flow channel; and a sensor configured to detect that the delivered liquid sample has reached a predetermined region between the measurement portion and the vent hole.

Even in such an embodiment, the stability of liquid delivery of the liquid sample introduced through the pipette tip can be ensured.

### Brief Description of the Drawings

[Fig. 1] Fig. 1 is a diagram showing a schematic configuration of an analysis system according to an embodiment.
[Fig. 2] Fig. 2 is a diagram illustrating a mode of use of a pipette tip according to the embodiment.
[Fig. 3] Fig. 3 is a diagram illustrating a microfluidic chip according to the embodiment.
[Fig. 4] Fig. 4 is a diagram illustrating a bubble removal structure provided in a flow channel of the microfluidic chip according to the embodiment.
[Fig. 5] Fig. 5 is a diagram illustrating an embodiment enabling tilted use and vertical use of the microfluidic chip according to the embodiment.
[Fig. 6] Fig. 6 is a diagram illustrating a bubble removal structure in the embodiment enabling tilted use and vertical use of the microfluidic chip according to the embodiment.
[Fig. 7] Fig. 7 is a table showing an example of a control sequence for the dispensing pipette during testing of the analysis system according to the embodiment.
[Fig. 8] Fig. 8 is an example of a graph showing absorbance measurement results.
[Fig. 9] Fig. 9 is a diagram showing a schematic configuration of an analysis system according to Example 2.
[Fig. 10] Fig. 10 is a top plan view illustrating a microfluidic chip according to Example 2.
[Fig. 11] Fig. 11 is a table showing an example of a control sequence for the dispensing pipette in Example 2.
[Fig. 12] Fig. 12 is a top plan view showing an example of a microfluidic chip according to Example 4.
[Fig. 13] Fig. 13 is a top plan view showing another example of the microfluidic chip according to Example 4.
[Fig. 14] Fig. 14 is a diagram illustrating a microfluidic chip according to a modified example.

### Embodiments for Carrying Out the Invention

Hereinafter, one mode for carrying out the invention (hereinafter also referred to as one embodiment or an embodiment) will be described with reference to the drawings. The configurations of the following embodiments are illustrative, and the configurations of the microfluidic chip and analysis system disclosed in the present embodiment may be appropriately modified depending on various conditions. Unless otherwise stated, the configurations disclosed in the present embodiment are not intended to limit the technical scope of the invention solely to those configurations, and may be combined to the extent possible.

Furthermore, the drawings referenced in the following description merely schematically illustrate shapes, sizes, and positional relationships to an extent sufficient to understand the content of the present invention. That is, the present invention is not limited solely to the shapes, sizes, and positional relationships illustrated in each drawing. In addition, there may be portions where the dimensional relationships and ratios differ between drawings.

### [Embodiment 1]

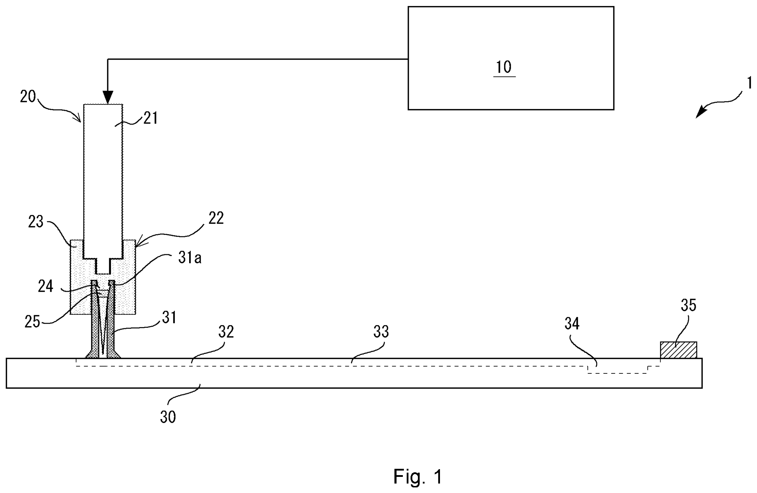

Fig. 1 is a diagram showing a schematic configuration of an analysis system 1 according to the present embodiment. The analysis system 1 illustrated in Fig. 1 is a system that measures and analyzes the reaction of a small amount of liquid sample such as a reagent or specimen introduced into a microfluidic chip 30 by a dispensing pipette 20. As shown in Fig. 1, the analysis system 1 is configured to include a control unit 10, a dispensing pipette (also simply referred to as a pipette) 20, and a microfluidic chip 30. The control unit 10 and the dispensing pipette 20 are connected via a predetermined interface.

The control unit 10 is a unit that controls the aspiration and dispensing of a small amount of liquid sample such as a reagent or specimen accommodated inside the dispensing pipette 20, and that controls the delivery of the liquid sample introduced in the form of droplets into the microfluidic chip 30. The introduction of a reagent or the like into the microfluidic chip 30 by the dispensing pipette 20 is also referred to as "spotting." The control unit 10 is, for example, a microcontroller unit comprising a processor such as a CPU (Central Processing Unit), a memory such as RAM (Random Access Memory), ROM (Read Only Memory), or flash memory, and a communication interface with external devices. The memory stores, for example, an operating system (OS), various programs, various tables, and the like. In the present embodiment, the delivery of the liquid sample in the microfluidic chip 30 is described as being controlled by the control unit 10 connected to the dispensing pipette 20; however, the control unit 10 and the dispensing pipette 20 may be configured integrally. That is, the dispensing pipette 20 may comprise, within its housing, a microcontroller chip or the like that executes the functions of the control unit 10. The dispensing pipette 20 may be configured to control the delivery of the liquid sample and the like in accordance with a program stored in advance in a memory or the like within the microcontroller chip.

The control unit 10 according to the present embodiment controls various parameters relating to the delivery of the liquid sample, such as flow rate, time, and volume, in accordance with the respective aspiration and dispensing operations of the dispensing pipette 20. In the microfluidic chip 30 into which the liquid sample has been introduced, it becomes possible to stably deliver the liquid sample through the flow channel 32 to the measurement portion 34, which is a region for analyzing and evaluating the components, properties, and the like of the liquid sample. In the analysis system 1 according to the present embodiment, stable liquid delivery can be provided even when the amount of the liquid sample used for testing or the like is small (for example, 5 to 20 µL).

The dispensing pipette 20 has a pipette body 21 that accommodates a liquid sample, and a pipette tip 22 attached to the distal end of the pipette body 21. The pipette tip 22 has a fixing portion 23 that secures the outer circumference of the pipette body 21 inserted therein, and a fitting portion 24. The fitting portion 24 is formed with, for example, a convex structure such as a barb structure protruding outward from the circumferential surface, and is fitted to a fitting structure 31a such as a fitting groove formed on the inner circumferential surface of the end portion of the connection portion 31 on the side where the pipette tip 22 is attached. The dispensing pipette 20 aspirates and dispenses a predetermined amount (for example, 5 to 20 µL) of the liquid sample accommodated in the pipette body 21 through the pipette tip 22.
The connection portion 31 may be integrated with the microfluidic chip 30, or may be a separate component from the microfluidic chip 30. The connection portion 31 may be detachable from the microfluidic chip 30.

The dispensing pipette 20, with the pipette tip 22 attached to the distal end of the pipette body 21, is held in the microfluidic chip 30 via a tubular connection portion 31 provided in the microfluidic chip 30. The connection portion 31 is a structural member for mounting and holding the dispensing pipette 20 containing the liquid sample in the microfluidic chip 30. On the inner circumferential surface of the end portion of the connection portion 31 on the side where the pipette tip 22 is attached, there is provided a fitting structure 31a such as a fitting groove that is fitted with a barb structure (convex structure) protruding outward from the circumferential surface of the fitting portion 24. As shown in Fig. 1, the distal end portion of the fitting portion 24 of the pipette tip 22 held in the microfluidic chip 30 via the connection portion 31 passes through the tubular interior space and extends into the region where the liquid sample is introduced.

The relative positioning of the distal end position of the pipette tip 22 with respect to the microfluidic chip 30 is appropriately adjusted by the engagement between the convex structure provided on the fitting portion 24 and the fitting structure 31a such as a fitting groove provided on the inner circumferential surface of the connection portion 31. Furthermore, in a state where the dispensing pipette 20 is mounted in the microfluidic chip 30 via the connection portion 31, as shown in Fig. 1, the outer circumferential surface of the connection portion 31 provided with the fitting structure 31a is in close contact with the inner circumferential surface of the pipette tip 22 at the portion where the convex structure of the fitting portion 24 is provided. The engagement between the convex structure provided on the fitting portion 24 and the fitting structure 31a provided on the inner circumferential surface of the connection portion 31 makes it possible to seal against leakage of air or the like when the liquid sample or the like is introduced into the microfluidic chip 30 through the mounted dispensing pipette 20. In the pipette tip 22, a contamination prevention filter 25 may be provided between the portion of the fitting portion 24 where the convex structure is provided and the distal end portion. The contamination prevention filter 25 is composed of, for example, a member that allows air to pass through while preventing the liquid sample from passing through.

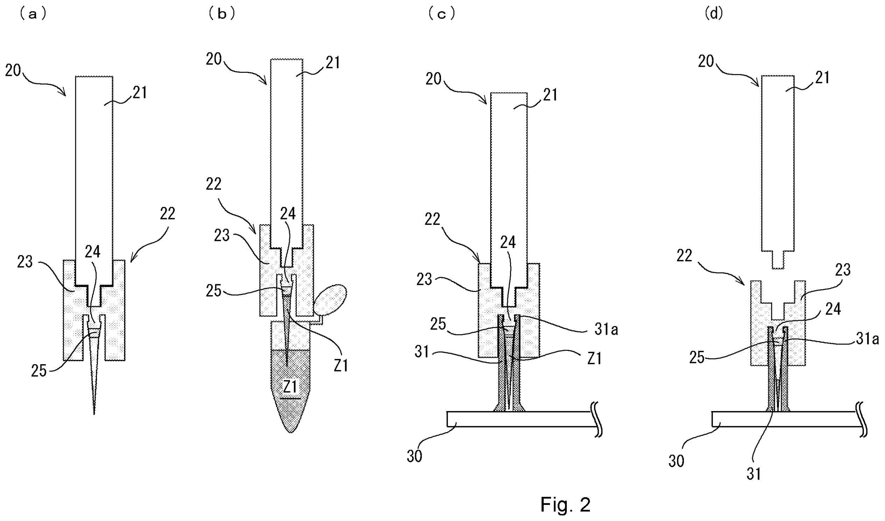

Fig. 2 is a diagram illustrating a mode of use of the pipette tip 22. Fig. 2 illustrates an example of a mode of use of the microfluidic chip 30 with a specimen Z1 collected through the pipette tip 22. As shown in Fig. 2, in the pipette tip 22, a contamination prevention filter 25 is provided in the interior space where the aspirated specimen Z1 is accommodated.

As shown in Fig. 2(a), prior to collection of the specimen Z1, the pipette body 21 of the dispensing pipette 20 is attached to the pipette tip 22. Via the fixing portion 23 of the pipette tip 22, the outer circumference of the pipette body 21 is secured to the pipette tip 22 by the internally inscribed fixing portion 23. The dispensing pipette 20 with the pipette tip 22 attached aspirates a predetermined amount (for example, approximately 5 to 20 µL) of the specimen Z1 contained in a container by an aspiration operation. The aspirated specimen Z1 is accommodated in the interior space at the distal end portion of the pipette tip 22, specifically between the tip and the contamination prevention filter 25 (Fig. 2(b)).

As shown in Fig. 2(c), the dispensing pipette 20 containing the specimen Z1 in the interior space of the distal end portion of the pipette tip 22 is mounted in the microfluidic chip 30 via the connection portion 31. The distal end position of the pipette tip 22 relative to the microfluidic chip 30 is positioned at a predetermined position by the engagement between the convex structure provided on the fitting portion 24 and the fitting structure 31a such as a fitting groove provided on the inner circumferential surface of the connection portion 31. The outer circumferential surface of the connection portion 31 provided with the fitting structure 31a is in close contact with the inner circumferential surface of the pipette tip 22 at the portion where the convex structure of the fitting portion 24 is provided, and the joint between the connection portion 31 and the pipette tip 22 is sealed.

Upon completion of the inspection and measurement of the specimen Z1 using the microfluidic chip 30, the pipette body 21 of the dispensing pipette 20 is detached from the attached pipette tip 22, as shown in Fig. 2(d). The pipette body 21 is withdrawn from the fixing portion 23 that secures the internally inscribed outer circumference, and the pipette tip 22, which is fitted to the connection portion 31 via the fitting portion 24, maintains its engaged state with the microfluidic chip 30.

When the pipette body 21 is withdrawn from the fixing portion 23, there may be cases where specimen Z1 that was not introduced into the microfluidic chip 30 remains in the interior space of the distal end portion of the pipette tip 22. The specimen Z1 remaining in the interior space of the distal end portion may flow back or scatter to the outside due to the negative pressure generated when the pipette body 21 is withdrawn, potentially causing environmental contamination of the surroundings. By providing the pipette tip 22 with a contamination prevention filter (contamination prevention filter 25), environmental contamination caused by the negative pressure generated when the pipette body 21 is withdrawn can be prevented in advance. The microfluidic chip 30 is discarded together with the pipette tip 22 fitted to the connection portion 31.

By operating the dispensing pipette 20 with the pipette tip 22 attached, it is possible to perform, for example, preprocessing of the specimen Z1, such as dilution of the specimen Z1 and mixing with a diluent, extraction of nucleic acids, and mixing with a reagent. These preprocessing steps may be appropriately combined. For example, by having the dispensing pipette 20 aspirate 10 µL (microliters) of the specimen Z1 through the pipette tip 22 and then further aspirating 90 µL of a diluent, the mixing of the specimen Z1 with the diluent through the aspiration operation can be promoted, enabling a 10-fold dilution of the specimen Z1 with the diluent. Furthermore, the pipette tip 22 may have, for example, a silica membrane capable of specifically binding to nucleic acids in the presence of a high salt concentration. By attaching the pipette tip 22 containing the silica membrane to the dispensing pipette 20, purified nucleic acids from the aspirated measurement specimen Z1 can be introduced into the microfluidic chip 30. Examples of measurement specimens Z1 include saliva, blood, nasal swab samples, and the like that have been treated with a lysis buffer used for nucleic acid extraction. After dispensing the above specimen into the microfluidic chip 30, the dispensing pipette 20 can wash the nucleic acids adsorbed to the silica membrane by aspirating and dispensing a washing solution containing ethanol or the like once again.

Furthermore, the pipette tip 22 may be configured to comprise, for example, a sponge or the like to which a reagent has been applied. During the aspiration and dispensing operations of the dispensing pipette 20 with the pipette tip 22 attached, it becomes possible to mix the reagent applied to the sponge or the like with the specimen Z1. For example, a sponge coated with calcium chloride is filled into the distal end portion of the pipette tip 22, blood or plasma anticoagulated with sodium citrate is aspirated, and the dispensing pipette 20 is mounted in the microfluidic chip 30. The dispensing pipette 20 can introduce calcium-supplemented blood or plasma into the microfluidic chip 30 by dispensing the blood or plasma aspirated into the pipette tip 22. Within the microfluidic chip 30, by pre-coating blood coagulation activation reagents such as tissue factor or contact factor activation reagents (kaolin, celite, etc.), it becomes possible to evaluate blood coagulation time within the microfluidic chip 30.

As described above, the liquid samples introduced into the microfluidic chip 30 through the pipette tip 22 include liquid samples such as reagents and specimens, reactive substances to be reacted with the liquid samples, washing solutions, and the like. The liquid sample is not particularly limited as long as it is a sample that can be passed through the microfluidic chip 30. Examples include liquid samples obtained from living organisms such as blood and urine, or dilutions thereof, extracts from living organisms such as plants and animals, naturally occurring water such as river water, seawater, and rainwater, cosmetics, washing solutions, waste liquids, and the like. The components in the sample are also not particularly limited, and examples include proteins, nucleic acids, low-molecular-weight compounds, sugars, and the like.

The microfluidic chip 30 is an instrument in which grooves forming a flow channel 32 for delivering a liquid sample introduced into a predetermined region of the connection portion 31 to the measurement portion 34 are formed. Such an instrument is produced, for example, by bonding a substrate having grooves serving as flow channels formed on its surface with a film material or another substrate using an adhesive or the like.

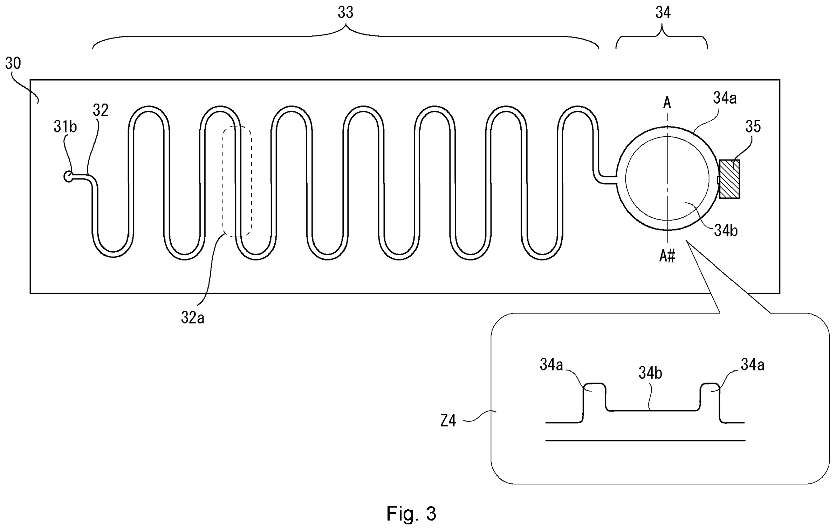

Fig. 3 is a diagram illustrating the microfluidic chip 30 according to the present embodiment. Fig. 3 illustrates a top plan view of the substantially rectangular microfluidic chip 30 as viewed from above. As shown in Fig. 3, a recessed region 31b, which is a depression for placing the liquid sample introduced in the form of droplets, is formed in the portion of the microfluidic chip 30 located opposite to the distal end portion of the fitting portion 24 of the dispensing pipette 20 held by the connection portion 31. A substantially circular measurement portion 34 is formed on the longitudinal end side of the microfluidic chip 30 facing the recessed region 31b, and a groove forming the flow channel 32 connecting the recessed region 31b and the measurement portion 34 is formed between the recessed region 31b and the measurement portion 34. The cross-sectional shape of the groove constituting the flow channel 32 may be any of concave, U-shaped, or V-shaped. The flow channel 32 is coated with, for example, a reaction substrate that reacts with the liquid sample introduced into the recessed region 31b by the dispensing pipette 20.

The flow channel 32 connecting the recessed region 31b and the measurement portion 34 is an elongated flow channel in which a plurality of substantially U-shaped flow channels 32 are connected as viewed from above, and constitutes, for example, a heating portion 33 where heating is performed to accelerate the reaction of the liquid sample. Below the flow channel 32 in which the heating portion 33 is formed, a heating mechanism such as a heater for heating the introduced liquid sample is provided. The liquid sample introduced into the flow channel 32 via the heating portion 33 can be heated in multiple stages according to the evaluation of its characteristics and properties.

The flow channel 32 constituting the heating portion 33 and the measurement portion 34 are provided with bubble removal structures 32a and 34a for capturing bubbles generated during the processing of the liquid sample introduced into the microfluidic chip 30. The measurement portion 34 is, for example, a region for analyzing and evaluating the components, properties, and the like of the liquid sample that reacts with a reaction substrate or the like. The measurement portion 34 may be provided with, for example, a stirring element for stirring the liquid sample delivered through the heating portion 33. The operation of the stirring element is controlled by, for example, a stirring mechanism utilizing magnetic force.

In the microfluidic chip 30, by providing the bubble removal structure 32a in the flow channel 32 constituting the heating portion 33, the mixing of bubbles generated during the processing of the liquid sample delivered to the measurement portion 34 can be suppressed. Furthermore, by also providing the bubble removal structure 34a in the measurement portion 34, interference of bubbles generated during the stirring process of the liquid sample introduced into the measurement portion 34 with the inspection and evaluation of the liquid sample can be suppressed. In the microfluidic chip 30, the stability of liquid delivery of the introduced liquid sample can be enhanced by suppressing the mixing of bubbles.

For example, when PCR is performed using the microfluidic chip 30, the liquid sample introduced into the flow channel 32 is heated through the heating portion 33 in a temperature range of 90 to 98 degrees Celsius. Similarly, when the LAMP method is applied, the liquid sample introduced into the flow channel 32 is heated through the heating portion 33 in a temperature range of 60 to 70 degrees Celsius. In either case, it is preferable to allow the heated liquid sample to remain in the region where the heating portion 33 is provided for approximately 3 to 10 minutes in order to generate sufficient bubbles. Bubbles generated during the processing of the liquid sample are captured via the bubble removal structure 32a formed in the flow channel 32, and the liquid sample from which bubbles have been removed is introduced into the measurement portion 34. Furthermore, in the measurement portion 34 as well, by providing the bubble removal structure 34a, bubbles that have flowed in through the flow channel 32 can be captured. As shown in Fig. 3, since the microfluidic chip 30 is configured with an elongated flow channel in which a plurality of substantially U-shaped flow channels 32 are connected in a meandering manner, the contact area between the flow channel wall surface in the heating portion 33 and the introduced liquid sample can be relatively increased, thereby enhancing the degree of bubble generation. Furthermore, in the region of the heating portion 33, by providing a plurality of repeating structures for capturing bubbles, such as a meandering flow channel 32, a bubble removal structure 32a, a meandering flow channel 32, and a bubble removal structure 32a, the bubble capture rate can be improved.

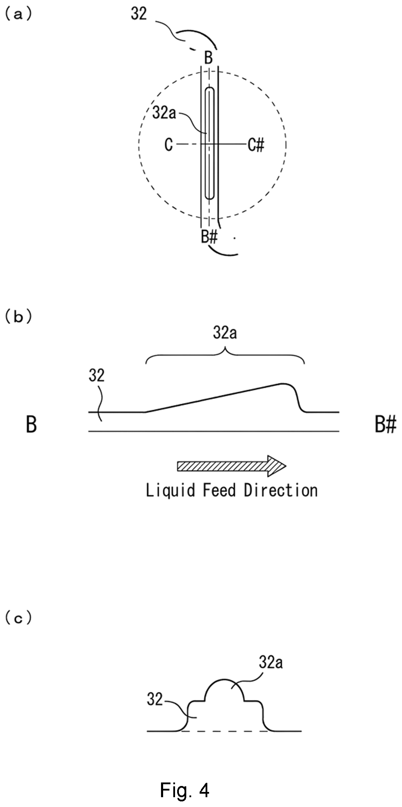

Fig. 4 is a diagram illustrating the bubble removal structure 32a provided in the flow channel 32. Fig. 4(a) illustrates an enlarged top plan view of a partial region of the flow channel 32 in which the bubble removal structure 32a is formed. Fig. 4(b) illustrates a cross-sectional view taken along line B-B# in Fig. 4(a), and Fig. 4(c) illustrates a cross-sectional view taken along line C-C# in Fig. 4(a). As shown in Fig. 4(a), in the region of the heating portion 33, the bubble removal structure 32a is formed in a linear flow channel 32 connecting flow channels that meander in a substantially U-shaped manner.

As shown in Figs. 4(b) and 4(c), the bubble removal structure 32a is formed as a convex structure protruding toward the surface side (the side where the connection portion 31 is provided) of the microfluidic chip 30 in the region of the heating portion 33. The protrusion height of the convex structure protruding toward the surface side of the bubble removal structure 32a is inclined linearly such that the protrusion height relatively increases in the flow direction from the upstream side to the downstream side of the flow channel 32. By providing such a structure, it becomes possible to enhance the accumulation efficiency of bubbles generated during the heating process.

Returning to Fig. 3, callout Z4 illustrates a cross-sectional view of the measurement portion 34 taken along line A-A#. As shown in callout Z4, the bubble removal structure 34a is formed as a convex structure protruding toward the surface side of the microfluidic chip 30 at the peripheral portion of the measurement portion 34. On the inner side where the bubble removal structure 34a is formed, a flat portion 34b is formed, which is a measurement region for measuring a property such as turbidity and absorbance of the inflowing liquid sample. The bubble removal structure 34a provided in the measurement portion 34 surrounds the flat portion 34b, protrudes toward the surface side, and is formed at the peripheral portion.

Regarding the removal of bubbles generated during the processing of the liquid sample introduced into the microfluidic chip 30, modes of use are envisaged in which the microfluidic chip 30, having the flow channel 32 formed therein for introducing the liquid sample, is used at an inclined angle relative to a horizontal plane, or in a vertically inverted orientation. For example, these modes include tilted use, in which the short side of the microfluidic chip 30 on which the connection portion 31 is provided faces vertically downward and the short side on which the measurement portion 34 is provided faces upward, with an inclined angle relative to the horizontal plane, and vertical use, in which the chip is vertically inverted. In such modes, the liquid sample introduced into the microfluidic chip 30 is delivered through the flow channel 32 from the lower side to the upper side in the vertical direction. The structure for capturing bubbles differs from the configuration in which the microfluidic chip 30 is installed horizontally, as described with reference to Figs. 3 and 4.

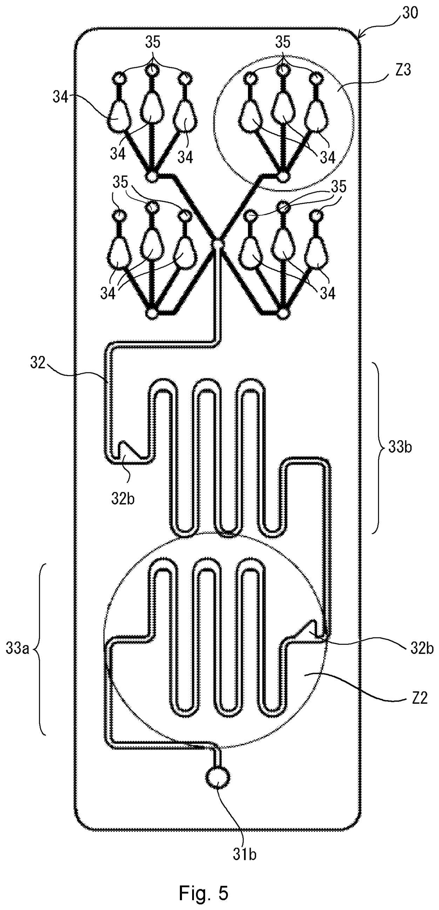

Fig. 5 is a diagram illustrating the microfluidic chip 30 in a form that enables the above-described tilted use and vertical use. Fig. 5 illustrates a form of the microfluidic chip 30 that enables multi-channel (3 channels × 4 blocks) reaction measurement. The microfluidic chip 30 of Fig. 5 is formed with flow channels 32 extending from the recessed region 31b to each of the measurement portions 34 provided in multiple channels, and heating portions 33a and 33b are provided in two stages on the lower side (recessed region 31b side) and the upper side (measurement portion 34 side) of the flow channel 32, respectively. Each of the heating portions 33a and 33b enables heating of the liquid sample introduced into the flow channel 32 at an individual temperature. The flow channel 32 connecting the heating portion 33a and the heating portion 33b, and the flow channel 32 connecting the heating portion 33b and the multi-channel measurement portions 34, are each provided with a bubble removal structure 32b for capturing bubbles generated during the processing of the introduced liquid sample.

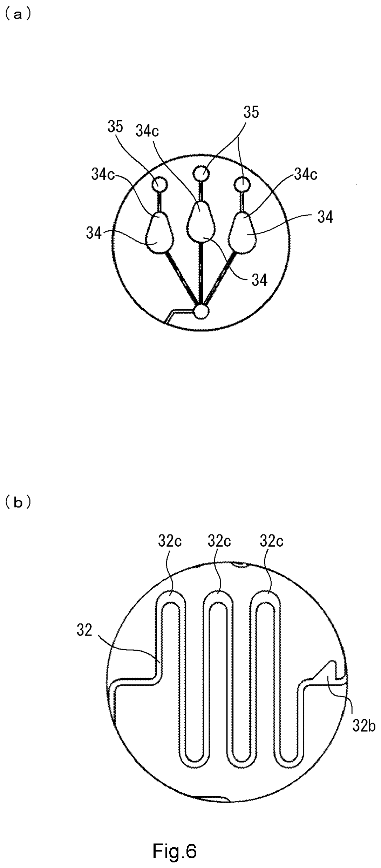

Fig. 6 is a diagram illustrating the bubble removal structure provided in the microfluidic chip 30 in a form that enables tilted use and vertical use. Fig. 6(a) is an enlarged view of the circled region Z3 in Fig. 5, illustrating the bubble removal structure 34c provided in the upper region of each measurement portion of the 3 channels. Fig. 6(b) is an enlarged view of the circled region Z2 in Fig. 5, illustrating the bubble removal structures 32b and 32c formed in the flow channel 32 of the heating portion 33b.

When the microfluidic chip 30 is tilted or oriented vertically, bubbles accumulate on the upper side in the vertical direction of the substantially U-shaped meandering flow channels 32 and on the upper side in the vertical direction of the measurement portion 34. For this reason, as shown in Fig. 6(b), in the substantially U-shaped meandering flow channels 32, the bubble accumulation efficiency can be enhanced by providing a bubble removal structure 32c that widens the flow channel space between the upper flow channel wall surface and the lower flow channel wall surface in the vertical direction. The same applies to the bubble removal structure 32b provided in the flow channel 32 connecting the heating portion 33a and the heating portion 33b. The bubble removal structure 32b can accumulate bubbles on the upper flow channel wall surface side by, for example, adopting a substantially triangular shape in which the upper flow channel wall surface in the vertical direction is expanded upward. Furthermore, in the measurement portion 34, by configuring the shape as viewed from the surface side of the measurement portion 34 into a substantially droplet shape, a bubble removal structure 34c that accumulates bubbles on the upper flow channel wall surface side in the vertical direction can be formed.

The measurement portion 34 is provided with a filter 35 that maintains the venting pressure of the flow channel 32 from the recessed region 31b to the measurement portion 34 within a predetermined pressure range. The filter 35 is provided, for example, in the measurement portion 34 at a location opposite to the inlet through which the liquid sample that has passed through the heating portion 33 flows in. Since the filter 35 allows air to pass through while preventing the liquid sample from passing through, the venting pressure between the recessed region 31b and the measurement portion 34 is maintained within a predetermined range. That is, in the measurement portion 34, when the liquid sample reaches the filter 35 provided at the location opposite to the inlet through which the liquid sample flows in, the delivery of the liquid sample through the flow channel 32 from the recessed region 31b to the measurement portion 34 is stopped.

In the form of the microfluidic chip 30 shown in Fig. 6 that enables multi-channel inspection and testing, it is required to introduce the liquid sample uniformly into each of the plurality of measurement portions. For example, it is required to introduce the liquid sample uniformly into the three measurement portions 34 of each block arranged on the upper side in the vertical direction, and into the three measurement portions 34 of each block arranged on the lower side in the vertical direction. By equipping the measurement portion 34 with the filter 35, the delivery of the liquid sample can be stopped at the point when the liquid sample reaches the measurement portion 34. That is, since the delivery to the measurement portion 34 arranged on the lower side in the vertical direction can be stopped at the point when the liquid sample reaches that measurement portion, liquid delivery can be continued sequentially and continuously to each channel in the branched paths to which the liquid sample has not yet arrived. As a result, even in the microfluidic chip 30 having branched flow channels to a plurality of (for example, 10 or more) measurement portions 34, the liquid sample can be introduced uniformly into each measurement portion 34.

While the configuration in Fig. 3 and the like includes the filter 35, a sensor that detects that the delivered liquid sample has reached a predetermined region (for example, the measurement portion 34) may be provided in place of the filter 35. Examples of such sensors include a liquid sensor or electrode sensor that detects the liquid level, a pressure sensor that detects the venting pressure of the flow channel 32 from the recessed region 31b to the measurement portion 34, and the like.

The control unit 10 of the analysis system 1 detects, for example, using a liquid sensor or electrode sensor, that the liquid sample delivered through the flow channel 32 has reached a predetermined region. The control unit 10 may control the stopping of liquid delivery based on the reached state indicated by a binary status signal representing an active status or inactive status output from the liquid sensor or electrode sensor. For example, the dispensing pipette 20 is controlled to continue liquid delivery when the status signal output from the electrode sensor indicates an inactive status representing a state of non-arrival, and to stop liquid delivery otherwise. In the present embodiment, by using a liquid sensor or electrode sensor, it becomes possible to stop the delivery of the liquid sample at a target location (a desired location in the flow channel 32 of the microfluidic chip 30).

Furthermore, the control unit 10 of the analysis system 1 can, for example, detect the venting pressure of the flow channel 32 from the recessed region 31b to the measurement portion 34 using a pressure sensor, and control the stopping of liquid delivery at the stage when the detected pressure reaches a constant pressure (threshold value). In the present embodiment, by using a pressure sensor, it becomes possible to sense a constant pressure increase in the venting pressure of the flow channel 32 from the recessed region 31b to the measurement portion 34, and to stop liquid delivery to the microfluidic chip 30.

### (Example 1)

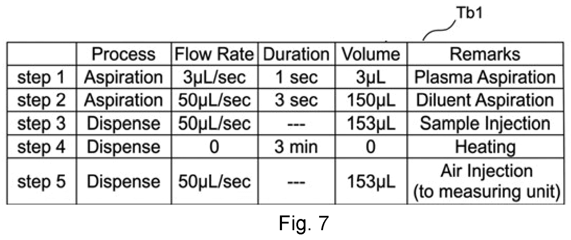

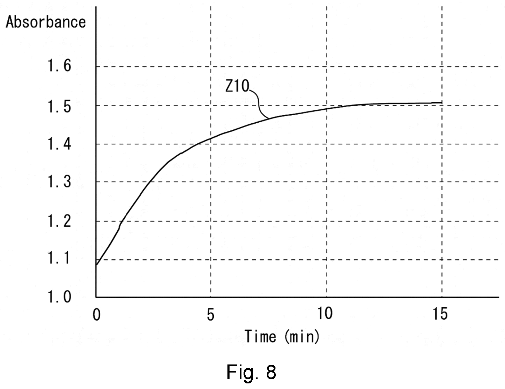

Next, the results of a liquid delivery test conducted using the analysis system 1 according to the present embodiment will be described. Fig. 7 is a table showing an example of a control sequence for the dispensing pipette 20 during the test, and Fig. 8 is an example of a graph showing the absorbance measurement results. In the liquid delivery test, plasma and a diluent were used as liquid samples, and the liquid samples were introduced into the microfluidic chip 30 using the dispensing pipette 20 with the pipette tip 22 attached. Subsequently, the delivery of the liquid sample was controlled, and the change in absorbance of the liquid sample delivered to the measurement portion 34 was measured.

As shown in Table Tb1 of Fig. 7, plasma was aspirated using the dispensing pipette 20 with the pipette tip 22 attached. The aspiration of plasma was performed for 1 second at a flow rate of 3 µL/second, aspirating a flow volume of 3 µL (step 1). The aspiration of the diluent was performed for 3 seconds at a flow rate of 50 µL/second, aspirating a flow volume of 150 µL (step 2). Thereafter, the dispensing pipette 20 with the pipette tip 22 attached was fixed to the connection portion 31 of the microfluidic chip 30, and measurement was commenced.

After the start of measurement, the diluted plasma specimen was introduced into the recessed region 31b through the pipette tip 22 of the dispensing pipette 20 fixed to the connection portion 31, and liquid delivery to the measurement portion 34 was commenced through the flow channel 32 formed in the microfluidic chip 30. The dispensing of the diluted plasma specimen was performed at a flow rate of 50 µL/second, dispensing the total volume (153 µL) (step 3).

The diluted plasma specimen was delivered from the recessed region 31b through the flow channel 32, reached the heating portion 33 set to a heating temperature of 37°C, and was heated. Three minutes after the cessation of liquid delivery in step 3, air inside the dispensing pipette was discharged through the dispensing pipette 20. The air was discharged at a flow rate of 50 µL/second, dispensing a flow volume of 153 µL (steps 4 and 5). Through the dispensing operation of step 5, the plasma specimen heated in the heating portion 33 provided in the flow channel 32 of the microfluidic chip 30 is delivered and injected into the measurement portion 34.

The measurement portion 34 is equipped with latex agglutination measurement beads and a stirring element (φ0.5 mm × length 1 mm). The plasma specimen injected into the measurement portion 34 is stirred at 500 rpm by the stirring element. After stirring, the change in absorbance at a wavelength of 600 nm was measured. The change in absorbance is measured, for example, as a change in transmittance using anti-D-dimer antibody-immobilized latex beads. The capacity of the measurement portion is 203 µL.

Fig. 8 shows an absorbance curve; the change in absorbance is the result of binding between the plasma specimen and the anti-D-dimer antibody immobilized on the latex beads, which is illustrated as a simulated reaction curve Z10. The vertical axis of the graph shown in Fig. 8 represents absorbance, and the horizontal axis represents time (minutes). As shown by the simulated reaction curve Z10, the absorbance (transmittance) of the plasma specimen due to the latex beads changes from approximately 1.1 to approximately 1.43 within 5 minutes from the start of measurement. During the 5-minute period from 5 to 10 minutes, the change in absorbance becomes gradual, transitioning from approximately 1.43 to approximately 1.5. After 10 minutes have elapsed, it was confirmed that the change in absorbance becomes a minute increase, remaining at approximately 1.5.

### (Example 2)

The microfluidic chip 30 of Example 1 was configured to maintain the venting pressure of the flow channel 32 from the recessed region 31b to the measurement portion 34 within a predetermined range by means of the filter 35 provided at the location in the measurement portion 34 opposite to the inlet through which the liquid sample flows in. In Example 2, the microfluidic chip 30 is provided with an air vent hole 36, which is a vent hole, in place of the filter 35, and a sensor 37 is installed in the flow channel 38 between the measurement portion 34 and the air vent hole 36. The sensor 37 is, for example, a liquid sensor or electrode sensor that detects that the delivered liquid sample has reached a predetermined region. Even with such a configuration, the control unit 10 of the analysis system 1 can control the stopping of the discharge of the liquid sample from the dispensing pipette 20 based on a binary status signal (detection signal) output from the sensor 37. As a result, in the embodiment of Example 2, it becomes possible to ensure the stability of liquid delivery in the microfluidic chip 30 while suppressing the component costs on the chip side and achieving accurate liquid volume control. In the embodiment of the analysis system 1 of Example 2, specimen measurement with improved accuracy becomes possible.

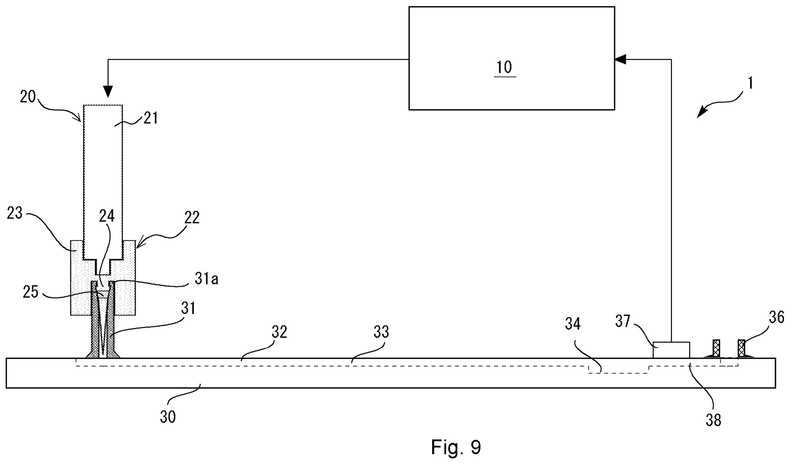

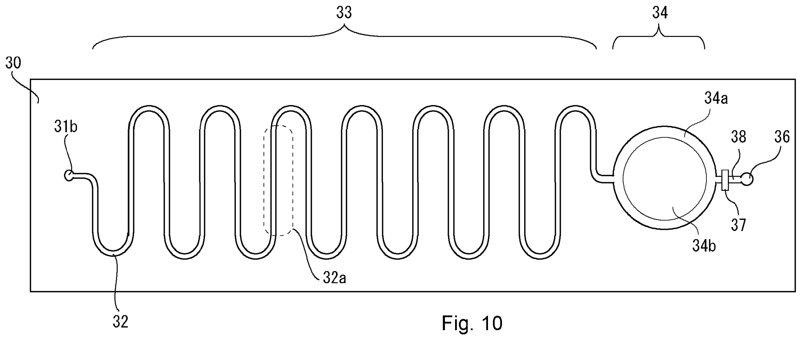

Fig. 9 is a diagram showing a schematic configuration of the analysis system 1 according to Example 2, and Fig. 10 is a top plan view illustrating the microfluidic chip 30 according to Example 2. As described above, the embodiment of Example 2 differs in that, in place of the filter 35, the microfluidic chip 30 is provided with an air vent hole 36, and a sensor 37 is installed in the flow channel 38 between the measurement portion 34 and the air vent hole 36. Furthermore, the control unit 10 of the analysis system 1 according to Example 2 differs in that it controls the delivery and stopping of the liquid sample discharged from the dispensing pipette 20 based on the binary status signal output from the sensor 37. Hereinafter, the analysis system 1 according to Example 2 will be described with a focus on the differences. The flow channel 32 is an example of a "first flow channel," and the flow channel 38 is an example of a "second flow channel." The first flow channel may have a storage portion, a preprocessing portion, an observation portion, and the like. The second flow channel may also have a waste liquid storage portion and the like.

As shown in Fig. 9, the binary status signal output from the sensor 37 is input to the control unit 10. For example, when the status signal output from the sensor 37 is an inactive status indicating that the liquid sample has not yet reached the flow channel 38 between the measurement portion 34 and the air vent hole 36, the control unit 10 controls the dispensing pipette 20 to continue discharging the liquid sample. On the other hand, when the status signal output from the sensor 37 is an active status indicating that the liquid sample has reached the flow channel 38 between the measurement portion 34 and the air vent hole 36, the control unit 10 controls the dispensing pipette 20 to stop discharging the liquid sample, and controls the stopping of liquid delivery in the microfluidic chip 30.

As shown in Figs. 9 and 10, the air vent hole 36 is formed on the longitudinal end side of the substantially rectangular microfluidic chip 30 facing the recessed region 31b. The sensor 37 is installed in the flow channel 38 between the measurement portion 34 and the air vent hole 36. The liquid sample delivered from the dispensing pipette 20 to the microfluidic chip 30 reaches the measurement portion 34 while pressing the air in the flow channel 32. Gas such as air present in the flow channel 32 that is pressed by the liquid sample is released to the outside of the microfluidic chip 30 through the air vent hole 36. When the discharge of the liquid sample from the dispensing pipette 20 continues, the liquid sample that has reached the measurement portion 34 arrives at the installation position of the sensor 37 installed in the flow channel 38 between the measurement portion 34 and the air vent hole 36. The sensor 37 detects the liquid sample that has arrived at the installation position and transitions the status signal from the inactive status to the active status. When the status signal transitions to the active status, the control unit 10 controls the dispensing pipette 20 to stop discharging the liquid sample. In the microfluidic chip 30 according to Example 2, the delivery of the liquid sample from the recessed region 31b through the flow channel 32 to the measurement portion 34 is stopped.

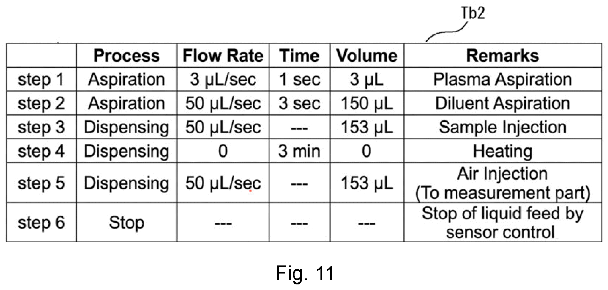

In the embodiment of Example 2 as well, a liquid delivery test was conducted in the same manner as in Example 1. Fig. 11 is a table showing an example of a control sequence for the dispensing pipette 20 in Example 2. In Example 2, since the sensor 37 that detects that the liquid sample has reached a predetermined region is provided, latex particles (50 µL) were enclosed in the heating portion 33 in advance. Since the latex particles enclosed in the heating portion 33 are enclosed in the elongated flow channel 32, they remain stably in the flow channel without displacement or scattering during storage and transportation of the microfluidic chip.

In Table Tb2 of Fig. 11, the processing of steps 1 through 3 is the same as in Example 1. That is, plasma (3 µL) was aspirated using the dispensing pipette 20 with the pipette tip 22 attached, followed by aspiration of the diluent (150 µL), and the dispensing pipette 20 with the pipette tip 22 attached was fixed to the connection portion 31 of the microfluidic chip 30, and measurement was commenced. After the start of measurement, the dispensing pipette 20 introduced the diluted plasma specimen into the recessed region 31b and commenced liquid delivery to the measurement portion 34 through the flow channel 32 formed in the microfluidic chip 30. The dispensing of the diluted plasma specimen was performed at a flow rate of 50 µL/second for approximately 3 seconds, dispensing a flow volume of 153 µL.

The diluted plasma specimen was delivered from the recessed region 31b through the flow channel 32, reached the heating portion 33 set to a heating temperature of 37°C, and was heated (step 4). At this time, the latex particles that had been enclosed in the heating portion 33 moved to the measurement portion 34 together with the plasma specimen. Then, three minutes after the cessation of liquid delivery in step 3, the diluted plasma specimen (153 µL) was discharged through the dispensing pipette 20 (step 5). The plasma specimen heated in the heating portion 33 is injected into the measurement portion 34, mixed with the latex particles, and the reaction is initiated. The sensor 37 installed in the flow channel 32 between the measurement portion 34 and the air vent hole 36 detects the plasma specimen that has reached that region, and the delivery of the plasma specimen in the microfluidic chip 30 is stopped (step 6). In Example 2, the delivery of the liquid sample in the microfluidic chip 30 may be controlled in combination with a pressure sensor, a valve, or the like.

As described above, in the analysis system 1 according to the present embodiment, the liquid delivery from the dispensing pipette 20 with the pipette tip 22 attached to the microfluidic chip 30 can be controlled using flow rate, time, volume, and the like as parameters. Furthermore, the delivery of the liquid sample that has reached the measurement portion 34 through the flow channel 32 formed in the microfluidic chip 30 can be stopped by the filter 35 or by using a binary output signal output from the sensor 37 such as a liquid sensor or electrode sensor. In the present embodiment, even when the amount of the liquid sample used for testing or the like is small, stable delivery of the liquid sample introduced through the flow channel formed in the microfluidic chip to a predetermined region can be provided.

### (Example 3)

An RNA extract was prepared from a nasal swab specimen using an RNA extraction kit (Loopamp Viral RNA Extraction Kit; Eiken Chemical). The dispensing pipette 20 aspirated the RNA extract (specimen) through the pipette tip 22 and introduced the aspirated RNA extract into the recessed region 31b of the microfluidic chip 30 to which it was fitted. The specimen was then delivered through the flow channel 32 to the heating portion 33 and stopped for 3 minutes. The heating portion 33 was heated to 65°C by a heater. After 3 minutes had elapsed, the dispensing pipette 20 was controlled and liquid delivery in the microfluidic chip 30 was commenced. The specimen heated to the predetermined temperature (65°C) via the heating portion 33 passed through the bubble removal structure 32a provided in the flow channel 32, and the specimen from which bubbles had been removed was introduced into reaction portion A (measurement portion 34, channel A) and reaction portion B (measurement portion 34, channel B). On reaction portions A, B, and C (measurement portion 34, channel C), LAMP method influenza test reagents (Loopamp Influenza B Virus Detection Reagent Kit; Eiken Chemical) were applied, with A for measurement, B for positive control, and C for negative control.

Each of the reaction portions A, B, and C was heated to 60°C. Downstream of the reaction portions A, B, and C, the flow channel 32 connected to the filter 35 continues further. The filter 35 allows air to pass through but does not allow liquid to pass through. By liquid delivery via the dispensing pipette 20, the reaction portions A, B, and C are filled with the specimen and reacted with the reagents applied to the respective reaction portions. The turbidity of the reaction portions A, B, and C was evaluated using a semiconductor laser with a wavelength of 600 nm. In the case of a positive result, an increase in turbidity is detected for reaction portions A and B.

### (Example 4)

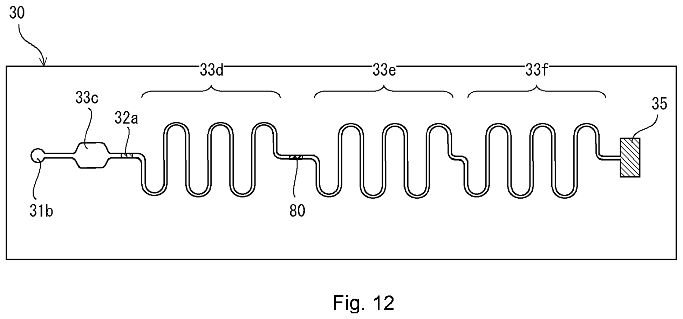

A DNA extract was prepared from a blood sample using a DNA extraction kit (DNeasy Blood & Tissue Kit; Qiagen). In the same manner as in Example 3, the dispensing pipette 20 aspirated the DNA extract (specimen) through the pipette tip 22 and introduced the aspirated DNA extract into the recessed region 31b of the microfluidic chip 30 to which it was fitted. The microfluidic chip 30 used in Example 4 is, for example, shown in Fig. 12. By controlling the dispensing operation of the dispensing pipette 20, the specimen was delivered through the flow channel 32 to the heating portion 33c and stopped for 3 minutes. The heating portion 33c was heated to 95°C by a heater. After 3 minutes had elapsed, the dispensing pipette 20 was controlled and liquid delivery in the microfluidic chip 30 was commenced. The specimen heated to the predetermined temperature (95°C) via the heating portion 33c passed through the bubble removal structure 32a provided in the flow channel 32, and the specimen from which bubbles had been removed was stopped at heating portion A (33d). After 1 minute had elapsed, the specimen was delivered to heating portion B (33e) and stopped for 30 seconds. Heating portion B (33e) was heated to 55°C by a heater. After 30 seconds had elapsed, the specimen was delivered to heating portion C (33f) and stopped. Heating portion B (33e) was heated to 72°C by a heater. Thereafter, the aspiration operation of the dispensing pipette 20 was controlled, and the specimen that had reached heating portion C (33f) was drawn back into the flow channel 32 of heating portion A (33d). By appropriately controlling the aspiration and dispensing operations of the dispensing pipette 20 and repeating the above cycle approximately 20 to 30 times, the PCR reaction proceeds. The PCR amplification in each cycle can be monitored as fluorescence intensity, for example, using a fluorescence detection portion 80 provided in the flow channel 32 between heating portion A (33d) and heating portion B (33e).

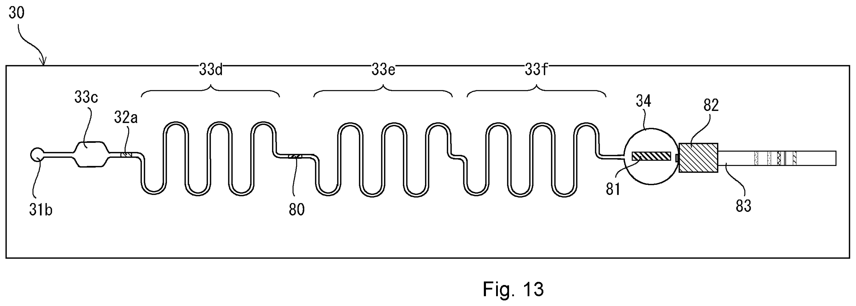

Furthermore, the detection of PCR amplification can be visually assessed by lateral flow, for example, by using a microfluidic chip 30 incorporating a nucleic acid chromatography test strip 83 as shown in Fig. 13. In that case, after nucleic acid amplification by PCR is performed with the dispensing pipette 20 as described above, the specimen is delivered by the dispensing pipette 20 to the reagent mixing portion (measurement portion 34) and stopped there for a predetermined period of time. In the reagent mixing portion (measurement portion 34), the specimen may be stirred via the inserted stirring element 81, or may be stopped for several minutes to allow natural diffusion of the specimen. In the reagent mixing portion (measurement portion 34), after the specimen is mixed with the reagent, it is further delivered and reaches the test strip reagent reaction portion 82. Thereafter, when the specimen reaches the nucleic acid chromatography test strip 83, the test results can be evaluated using nucleic acid chromatography.

### (Modification)

In Embodiment 1, the microfluidic chip 30 was described as a substantially rectangular instrument having grooves of the flow channel 32 formed therein for delivering a liquid sample from the recessed region 31b of the connection portion 31 for fixing the dispensing pipette 20 with the pipette tip 22 attached, to the measurement portion 34. For example, as disclosed in Japanese Patent Application Laid-Open No. 2021-159011, Japanese Patent Application Laid-Open No. 2019-088332, Japanese Patent Application Laid-Open No. 2018-014966, and the like, disk-shaped microchips are used in PCR, immunoassays, and the like. In the analysis system 1 of the modification, a disk shape is applied to the shape of the microfluidic chip 30 through which liquid delivery is performed by the dispensing pipette 20.

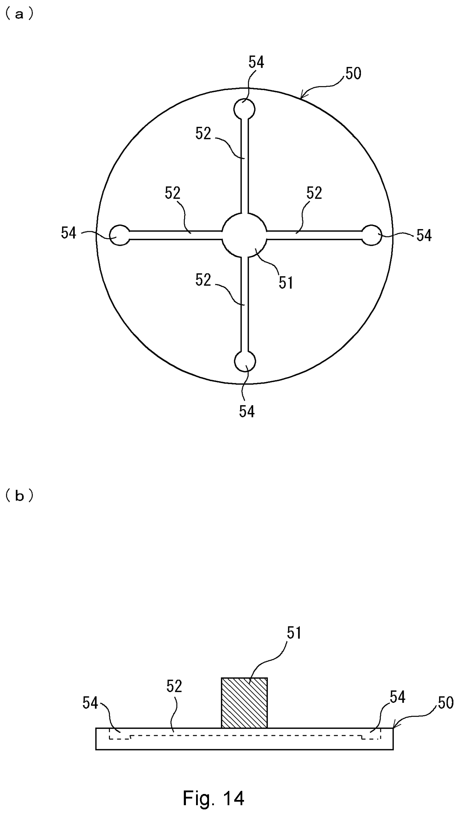

Fig. 14 is a diagram illustrating the microfluidic chip 50 according to the modification. Fig. 14(a) shows a top plan view of the microfluidic chip 50 according to the modification as viewed from above, and Fig. 14(b) shows a side view of the microfluidic chip 50 as viewed from the side. As shown in Figs. 14(a) and 14(b), the microfluidic chip 50 is disk-shaped, and a connection portion 51 for fixing the dispensing pipette 20 with the pipette tip 22 attached is formed at the center. Below the connection portion 51, for example, a reagent coating that reacts with the liquid sample, a plasma separation filter, a nucleic acid extraction filter, and the like can be installed.

Grooves of the flow channels 52 extending linearly from the central portion toward the peripheral portion where the connection portion 51 is provided are formed radially, and the grooves of the flow channels 52 are connected to measurement portions 54 formed at the peripheral portion. The form illustrated in Fig. 14(a) is an example in which grooves of four flow channels 52 are formed, intersecting perpendicularly in a cross shape at the central portion.

The dispensing pipette 20 with the pipette tip 22 attached is mounted to the connection portion 51 provided at the central portion of the disk-shaped microfluidic chip 50 in the same manner as in Examples 1 and 2, and the injection of the liquid sample into the flow channels 52 and the stepwise delivery to the measurement portions 54 are performed. Thereafter, by detaching the mounted dispensing pipette 20 from the connection portion 51 and advancing liquid delivery by centrifugation, two-stage liquid delivery becomes possible. That is, in the modified embodiment, by adopting the disk-shaped microfluidic chip 50, it becomes possible to perform two-stage liquid delivery: liquid delivery by the dispensing pipette 20 with controlled flow rate, time, and volume, and liquid delivery by centrifugation. In the modified embodiment, control through multistage precise liquid delivery becomes possible.

In the same manner as in Examples 1 and 2, in the modified embodiment as well, the analysis system 1 can perform complex liquid delivery control by combining it with valves, liquid sensors, electrode sensors, pressure sensors, and the like. In the liquid delivery of the modified embodiment, for example, mixing of a specimen with a reagent, plasma separation from a blood specimen, and nucleic acid extraction from a specimen as preparation for PCR, and the like are possible. In the case of mixing a specimen with a reagent, for example, a reagent is applied to the flow channel 52 and the specimen is injected up to the region where the reagent has been applied. Mixing then becomes possible by repeatedly aspirating and dispensing the specimen injected into the flow channel using the dispensing pipette 20. It is also possible to perform preprocessing such as plasma separation from whole blood as a pretreatment, and then perform liquid delivery by centrifugation.

### [Description of Reference Numerals]

1: Analysis system, 10: Control unit, 20: Dispensing pipette, 21: Pipette body, 22: Pipette tip, 23: Fixing portion, 24: Fitting portion, 25: Contamination prevention filter, 30, 50: Microfluidic chip, 31, 51: Connection portion, 31a: Fitting structure, 31b: Recessed region, 32, 38, 52: Flow channel, 32a, 32b, 34a, 34c: Bubble removal structure, 33, 33a, 33b, 33c, 33d, 33e, 33f: Heating portion, 34, 54: Measurement portion, 34b: Flat portion, 35: Filter, 36: Air vent hole, 37: Sensor

## Claims

1. A liquid sample analysis system comprising:
a pipette tip configured to aspirate and dispense a liquid sample;
a dispensing pipette having the pipette tip attached to a distal end thereof and having a space for accommodating the liquid sample aspirated through the pipette tip;
a microchip having a connection portion configured to hold the dispensing pipette with the pipette tip attached thereto, and having a first flow channel formed therein through which the liquid sample introduced from the dispensing pipette flows into a measurement portion for measuring a property of the liquid sample; and
a control unit connected to the dispensing pipette, configured to control a flow rate of the liquid sample aspirated and dispensed through the pipette tip, and configured to control delivery of the introduced liquid sample to the measurement portion through the first flow channel.

2. The liquid sample analysis system according to Claim 1, wherein the microchip comprises a filter configured to maintain a venting pressure during the delivery of the liquid sample through the first flow channel to the measurement portion within a predetermined range.

3. The liquid sample analysis system according to Claim 1, wherein the microchip comprises:
a vent hole connected to the measurement portion via a second flow channel; and
a sensor configured to detect that the delivered liquid sample has reached a predetermined region between the measurement portion and the vent hole,
wherein the control unit is configured to control the dispensing pipette to stop dispensing of the liquid sample accommodated in the dispensing pipette, based on a detection signal output from the sensor indicating that the delivered liquid sample has reached the predetermined region.

4. A microchip comprising:
a connection portion configured to hold a dispensing pipette having a pipette tip attached to a distal end thereof, the pipette tip being configured to aspirate and dispense a liquid sample, and the dispensing pipette having a space for accommodating the liquid sample aspirated through the pipette tip;
a flow channel through which the liquid sample introduced from the dispensing pipette held by the connection portion flows into a measurement portion for measuring a property of the liquid sample; and
a filter configured to maintain a venting pressure of the liquid sample delivered to the measurement portion through the flow channel within a predetermined range.

5. A microchip comprising:
a connection portion configured to hold a dispensing pipette having a pipette tip attached to a distal end thereof, the pipette tip being configured to aspirate and dispense a liquid sample, and the dispensing pipette having a space for accommodating the liquid sample aspirated through the pipette tip;
a first flow channel through which the liquid sample introduced from the dispensing pipette held by the connection portion flows into a measurement portion for measuring a property of the liquid sample;
a vent hole connected to the measurement portion via a second flow channel; and
a sensor configured to detect that the delivered liquid sample has reached a predetermined region between the measurement portion and the vent hole.
